# EUROPEAN PATENT SPECIFICATION

(11) **EP 3 793 645 B1**
(45) Date of publication and mention of the grant of the patent: **01.07.2026**
(21) Application number: 19803801.0
(22) Date of filing: 14.05.2019
(51) Int. Cl.: A61M 5/20, A61M 5/44, A61M 5/24, A61M 5/315

(54) **SYSTEMS FOR DELIVERING A SUBSTANCE TO A LIVING BEING**
SYSTEME ZUR ABGABE EINER SUBSTANZ AN EIN LEBEWESEN
SYSTÈMES POUR ADMINISTRER UNE SUBSTANCE À UN ÊTRE VIVANT

(30) Priority: 15.05.2018 US 201862671670 P
(43) Date of publication of application: 24.03.2021
(73) Proprietor: SHL Medical AG, 6300 Zug (CH)
(72) Inventor: DAHMANI, Alexander, New York, NY 10016 (US); SCHWARTZENTRUBER, Jared, New York, NY 10025 (US)
(86) International application number: PCT/US2019/032104
(87) International publication number: WO 2019/222140

(56) References cited:
- WO-A1-2017/156523
- WO-A2-2008/081444
- WO-A2-2008/114224
- US-A1- 2007 023 034
- US-A1- 2007 270 744
- US-A1- 2008 312 604
- US-A1- 2017 049 965
- US-A1- 2018 193 564
- US-A1- 2018 236 181

## Description

### CROSS-REFERENCE TO RELATED APPLICATION(S)

This application claims the benefit of U.S. Provisional Patent Application No. 62/671,670 filed May 15, 2018, entitled SYSTEMS AND METHODS FOR DELIVERING A SUBSTANCE TO A LIVING BEING.

### TECHNICAL FIELD

The present application relates to systems and methods for delivering a substance into a patient. In particular, the present application relates to systems and methods for delivering a dose of a substance into an object and subsequently verifying that the object was a living being.

### BACKGROUND

As drug delivery devices gain new functionalities (e.g., sensors and connectivity), they are being transformed into medication adherence monitoring devices. For the first time, these devices can provide medication consumption data outside of a clinical environment. Further, as health care professionals begin to make treatment and business decisions based on the medication consumption data, validity and accuracy of such data are becoming ever more important.

US2017/049965, US2007/023034, US 2008/0312604, WO2017/156523 and WO2008/081444 describe injection systems that may include sensors.

### SUMMARY

The scope of protection is defined by the claims, to which reference should now be made.

### BRIEF DESCRIPTION OF THE DRAWINGS

FIG. 1 illustrates an example of a drug delivery device for delivering a dose of a substance to a patient in accordance with the disclosed embodiments.
FIG. 2 illustrates various hardware components of the drug delivery device of FIG. 1.
FIG. 3 illustrates an example of a drug delivery device 100 in accordance with the disclosed embodiments.
FIGS. 4-7 illustrate additional examples of arrangements for sensing surfaces in accordance with the disclosed embodiments.
FIGS. 8-10 are example graphs illustrating temperature measured by temperature sensor 106 before, during, and after contact sensor 104 is in contact with an object.
FIG. 11 illustrates an example of a process 1100 for delivering a substance to a living being by drug delivery device 100 in accordance with the disclosed embodiments

### DETAILED DESCRIPTION

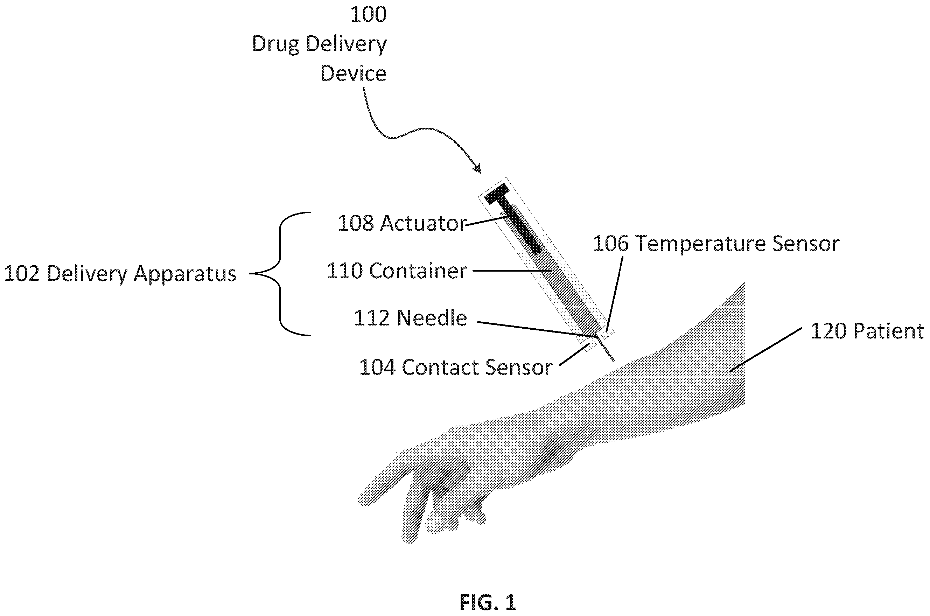

FIG. 1 illustrates an example of a drug delivery device 100 for delivering a dose of a substance to a patient 120 in accordance with the disclosed embodiments. As shown in FIG. 1, drug delivery device 100 may include a delivery apparatus 102, a contact sensor 104, and a temperature sensor 106. Further as shown in FIG. 1, delivery apparatus 102 may include an actuator 108, a container 110, and a needle 112.

### Overview

In the example of FIG. 1, patient 120 or another person (e.g., a health care professional) is attempting to use drug delivery device 100 to deliver a single dose of a substance (e.g., vaccine, medication) in container 110 into an arm of patient 120 via needled 112. Here, patient 120 may insert needle 112 of drug delivery device 100 into the arm such that contact sensor 104 comes in contact with the arm when needle 112 is properly inserted (e.g., to a correct depth and/or in a correct orientation). Contact sensor 104, after determining that it is indeed in contact with an object (i.e., patient 120), activates delivery apparatus 102. In response, delivery apparatus 102, using actuator 108, expels a single dose of the substance in container 110 via needle 112. In one example, container 110 may be a syringe that includes a plunger, and actuator 108 may expel the substance via needle 112, for example, by pushing on the plunger.

Temperature sensor 106 may be arranged to measure temperature at a location proximate to contact sensor 104 and/or to a location where needle 112 would puncture the object. For example, temperature sensor 106 may be disposed adjacent, or substantially proximate (e.g., within ~1 cm of each other), to contact sensor 104 and/or a location where needle 112 would puncture the object.

Additionally, temperature sensor 106 may be configured to measure the temperature before and while contact sensor 104 comes in contact with an object (i.e., patient 120 in FIG.1). Thus, temperature sensor 106 may be capable of measuring ambient temperature (e.g., when contact sensor 104 is not in contact with the object) as well as temperature of the object while contact sensor 104 is in contact with the object ("contact temperature"). In some embodiments, temperature sensor 106 may be further configured to measure the temperature after contact sensor 104 loses contact (i.e., disengages) with the object ("post-contact temperature"). In some embodiments, the ambient temperature and/or post-contact temperature may be used to calibrate temperature sensor 106 and/or the measured contact temperature.

As used herein, the terms "ambient temperature" refer to temperature measured by temperature sensor 104 before contact sensor 104 comes in contact with an object. The terms "contact temperature" refer to temperature measured by temperature sensor 106 while contact sensor 104 is in contact with an object. The terms "post-contact temperature" refer to the temperature measured by temperature sensor 106 after contact sensor 104 disengages with the object. The measured temperature may be considered post-contact temperature (i) for a predetermine amount of time after contact sensor 104 disengages, (ii) until the temperature stabilizes (e.g., variance is below a threshold value), and/or (iii) until the measured temperature reaches, or become close to, a previously-measured ambient temperature or a predetermined temperature. As used herein, the terms "measured temperature" refers to ambient temperature, contact temperature, and post-contact temperature as a collective.

After the substance is delivered, the measured temperature may be used to determine whether the object to which the substance was delivered ("target object") was a living being, as opposed to an inanimate object. The measured temperature may be further used to identify an area of the target object to which the substance was delivered ("delivery area/site"). For example, contact temperature that is maintained between 86°F and 94°F (in a 75°F environment) may indicate that the target object was likely a human being. Similarly, contact temperature that is initially at 92°F and decreases to 89°F after ~10 seconds may also indicate that the target object was likely a human being and that the delivery area was likely an abdomen or upper thigh area (shortly after the clothing was removed from the area). Contact temperature that increases rapidly (e.g., from 62°F to 71°F in 15 seconds) may also indicate that the target object was likely a human being and that the delivery area was likely applied with some ice prior to the delivery of the dose. The inventor(s) of the present application have been determined that the rate at which human skin temperature rises in room temperature after applying ice may be substantially constant. In contrast, contact temperature that is maintained at or around room temperature (e.g., 75°F) may indicate that the target object was likely an inanimate object (e.g., wet paper towel or an orange). The inventor(s) also determined that the magnitude of temperature decrease or increase may also be affected by the person's baseline skin temperature and the ambient temperature.

In some embodiments, drug delivery device 100 may determine whether the target object was a living being or an inanimate object. Alternatively, or additionally, drug delivery device 100 may transmit data (e.g., measured temperature, contact sensor data) to an external device, and the external device may determine whether the target object was a living being or an inanimate object.

Capability to distinguish, after delivering a dose, whether the target object was a living being or an inanimate object is important for various reasons. For example, such a capability may be used to determine whether a patient is adhering to a prescribed regimen in situations where adherence is critical (e.g., a clinical study) and/or in situations where there are reasons for patients to "fake" an injection (e.g., psychiatric patients or "professional" patients being compensated for taking a medication). In another example, such a capability may be used to improve accuracy in determining whether a dose is delivered successfully to a patient. In particular, such a capability may be used to distinguish, for example, an accidental discharge of the drug that may result when an injection device is dropped. In yet another example, such a capability may be used to distinguish a syringe priming process and/or a test/practice injection process (e.g., into a dummy injection pad).

Confirming that an injection device is in contact with a patient is important for preventing premature activation and pausing incomplete dosing events. But, while pressure and capacitive sensors may be reliable for detecting contact with an object, they can easily be activated by objects other than patient skin. For example, a patient may be able to activate a drug delivery device using a moist paper towel or an orange. Temperature is a more reliable metric for differentiating between patient tissue and other objects. But, temperature may not be a reliable activation mechanism for a drug delivery device because skin temperature can vary, especially if a patient applies ice to the injection site in order to reduce pain. In contrast, skin temperature can be used to validate drug delivery events that are initiated by other mechanisms. In particular, a constant temperature over the course of a drug delivery event in the range of 30-35C is highly correlated to patient tissue. A rising skin temperature over the course of a drug delivery event would be correlated to an iced injection site. Thus, a patient sensing system that uses a contact sensor to activate a drug delivery device and a temperature sensor to validate the drug delivery event provides the best of both worlds: reliable device activation and reliable dosing data.

In some embodiments, drug delivery device 100 may be an inhalation device and delivery apparatus 102, after being activated, may release inhalant stored in container 110. In some embodiments, the inhalation device would be in contact with the user's lips and/or inner mouth. The temperature recorded before, during and after activation of the inhalation device would indicate whether the device was properly oriented with respect to the user's mouth.

### Delivery Apparatus

In FIG. 1, delivery apparatus 102 may be configured to expel a dose of the substance contained in container 110 via needle 112 (e.g., into the arm of patient 120 if needle 120 is inserted in the arm) after delivery apparatus 102 is activated and/or in response to delivery apparatus 102 being activated. In some embodiments, delivery apparatus 102 may cease expelling the substance after delivery apparatus 102 is deactivated and/or in response to delivery apparatus 102 being deactivated.

Delivery apparatus 102 may be activated/deactivated based on a signal from other components of drug delivery device 100. For example, delivery apparatus 102 may be activated in response to a signal generated by contact sensor 104 indicating that contact sensor 104 is in contact with an object. In another example, delivery apparatus 102 may be activated in response to a signal from a processor indicating that contact sensor 104 is in contact with an object. In yet another example, delivery apparatus 102 may be activated in response to a signal indicating that contact sensor 104 is in contact with an object and an input from a user indicating that the user is ready to receive the dose. Correspondingly, delivery apparatus 102 may be deactivated in response a signal generated by contact sensor 104 indicating that contact sensor 104 is no longer in contact with the object. In another example, delivery apparatus 102 may be deactivated in response to a signal from a processor indicating that contact sensor 104 is no longer in contact with an object.

In some embodiments, delivery apparatus 102, after being activated, may deactivate itself (or be deactivated by another component) (i) after a predetermined amount of time, (ii) after determining that a predetermined amount of the substance (e.g., a dose) is expelled, and/or (iii) after determining that an amount of the substance contained in container 110 has reached a predetermined value. For example, delivery apparatus 102 may deactivate itself after 5 seconds, after 1mL of the substance is expelled from container 110, and/or after determining that the volume of the substance in container 110 reached 1mL. In some embodiments, delivery apparatus 102 may deactivate itself (or be deactivated by another component) after a set of predetermined conditions are met. Such conditions may be met, for example, (i) when contact temperature measured by temperature sensor 106 exhibits an unexpected trend (e.g., sudden drop/increase in temperature), (ii) when the substance is expelled at an unexpected (e.g., too fast for needle 112), and/or (iii) when contact pressure sensed by contact sensor 104 fluctuates in an unexpected manner (e.g., too much vibration). Meeting these conditions may indicate, for example, that drug delivery device 100 is malfunctioning and/or being used improperly.

In some embodiments, delivery apparatus 102 may be configured to perform additional processes upon activation and/or deactivation. As an example, in some embodiments, needle 112 of delivery apparatus 102 may be retractable. In this example, needle 112 may be in a retracted position initially such that, when contact sensor 104 comes into contact with an object, needle 112 is not in contact with the object. After being activated, delivery apparatus 102 may be configured to extend needle 112 so as to puncture the object and deliver the substance. Correspondingly, after being deactivated, delivery apparatus 102 may be configured to retract needle 112 to disengage with the object. In some embodiments, after being activated, delivery apparatus 102 may be configured to rapidly extend needle 112 so as to puncture the object, deliver a dose of the substance, and rapidly retract needle 112 to disengage with the object after the dose is delivered.

In FIG. 1, delivery apparatus 102 may deliver a dose of the substance in container 110 using actuator 108. In some embodiments, container 110 may include a tubular structure containing the substance and a plunger at an end opposite to needle 112. In these embodiments, actuator 108 may include a linear actuator arranged to push the plunger into the tubular structure such that the substance is expelled through needle 112. In some embodiments, the linear actuator may include a hollow drive shaft and a lead screw may be disposed inside and connected to the hollow drive shaft.

In some embodiments, delivery apparatus 102 may include one or more sensors for detecting an amount of the substance expelled from container 110 and/or an amount of the substance remaining in container 110.

In some embodiments, drug delivery device 100 may be a reusable/recyclable device. In these embodiments, container 110 may include a sufficient amount of the substance for a plurality of doses, and needle 112 may be replaceable and/or cleanable. Alternatively, or additionally, container 110 may be replaceable (e.g., when container 110 no longer contains sufficient amount of the substance for a dose). In some embodiments, container 110 and needle 112 may be mechanically coupled such that both parts can be replaced simultaneously. For example, needle 112 may be a part of a replaceable container 110.

As discussed above, in some embodiments, needle 112 may be retractable. In these embodiments, needle 112 may be manually extended and/or retracted. Alternatively, or additionally, needle 112 may extend and/or retract automatically (e.g., in response to an input from a user or when delivery apparatus 102 is activated/deactivated). In some embodiments, needle 112 may rapidly extend in a manner that can puncture, for example, the arm of patient 120. In some embodiments, needle 112 may have a length and arranged such that an insertion depth of needle 112 is in a predetermined range (e.g., between 4mm-12mm).

The processes above are described as being performed by delivery apparatus 102 (e.g., activation/deactivation of delivery apparatus 102, extending/retraction of needle 112, actuating actuator 108). However, in some embodiments, the processes may be at least partially performed, or enabled, by one or more processors that may be internal and/or external to delivery apparatus 102. That is, one or more processors may be configured to perform the above-described processes at least partially and/or enable delivery apparatus 102 to perform them. Further, one or more of such processors may be shared among components of drug delivery device 100.

### Contact Sensor

In FIG. 1, contact sensor 104 is used to detects when contact sensor 104 comes into contact with an object, such as an arm of patient 120. In particular, contact sensor 104 may be arranged relative to other components of drug delivery device 100 such that contact sensor 104 comes in contact with an object after needle 112 is positioned properly for insertion into the object. Further, contact sensor 104 may be configured to activate delivery apparatus 102 after determining that contact sensor 104 is in contact with an object, thereby causing delivery apparatus 102 to expel the substance contained in container 110 into the object. For example, after determining that contact sensor 104 has come in contact with an object, contact sensor 104 may transmit (or cause a processor to transmit) a signal to actuator 108 of delivery apparatus 102. In some embodiments, contact sensor 104 may further determine whether the contact with the object is proper or not. In these embodiments, contact sensor 104 may be configured to activate delivery apparatus 102 after determining that contact sensor 104 is in in contact with an object and that the contact is also proper.

Correspondingly, contact sensor 104 may be configured to deactivate delivery apparatus 102 after determining that contact sensor 104 is no longer in contact with a target object. In embodiments where contact sensor 104 is further configured to determine whether the contact with the object is proper, contact sensor 104 may be configured to deactivate delivery apparatus 102 after determining that contact sensor 104 is no longer in contact with an object or determining that that the contact is improper.

Furthermore, contact sensor 104 may generate data that can provide context to the temperature data measured by temperature sensor 106 ("temperature context data"). In particular, data generated by contact sensor 104 may be used to determine which portion of temperature data measured by temperature sensor 106 is ambient temperature (i.e., temperature before contact sensor 104 is in contact with an object), which portion is contact temperature (i.e., temperature while contact sensor 104 is in contact with the object), and which portion is post-contact temperature (i.e., temperature when contact sensor 104 is no longer in contact with the object).

In some embodiments, contact sensor 104 may be a mechanical sensor that measures pressure applied to a sensing surface. In these embodiments, contact sensor 104 (or a processor(s)) may determine that contact sensor 104 is in contact with an object by determining whether the measured pressure at the sensing surface is above a threshold value and/or maintained above the threshold value for a predetermined amount of time. Correspondingly, contact sensor 104 may determine that contact sensor 104 is not in contact with an object by determining whether the measured pressure at the sensing surface is below a threshold value and/or maintained below the threshold value for a predetermined amount of time.

In some embodiments, contact sensor 104 may be an electrical sensor that measures conductivity between points on a sensing surface(s) that is in contact with a target object (i.e., patient tissue). In these embodiments, contact sensor 104 (or a processor(s)) may determine that contact sensor 104 is in contact with the target object by determining whether the measured conductivity is above a threshold value and/or maintained above the threshold value for a predetermined amount of time. Correspondingly, contact sensor 104 may determine that contact sensor 104 is not in contact with an object by determining whether the measured conductivity at the sensing surface is below a threshold value and/or maintained below the threshold value for a predetermined amount of time.

The threshold value(s) may be predetermined or dynamically determined, for example, based on data collected from a plurality of drug delivery devices and/or based on data previously collected from drug delivery device 100.

In some embodiments, contact sensor 104 may determine that contact sensor 104 is or is not in contact with the target object when data generated by contact sensor 104 (e.g., data indicative of conductivity or pressure) satisfies one or more predetermined criteria. For example, contact sensor 104 may determine that contact sensor 104 is not in contact with the target object (or that the contact is improper) when the detected pressure/conductivity has a variance above a threshold variance value (e.g., even when the pressure is above the threshold value). In another example, contact sensor 104 may determine that contact sensor 104 is no longer in contact with the target object (or that the contact is improper) when the detected pressure/conductivity suddenly changes by a predetermined amount/percentage (e.g., even when the pressure/conductivity after the change is above the threshold value).

In some embodiments, contact sensor 104 may further include an inertial sensor (e.g., accelerometer, gyroscope), a directional sensor (e.g., magnetometer), and/or an image sensor to determine whether drug delivery device 100 is in a proper orientation/position relative to a target object. Such a sensor(s) may be used to determine whether the contact with a target object is proper or not.

In some embodiments, data generated by contact sensor 104 (e.g., raw data indicative of conductivity or pressure) may be provided to a processor or other components in drug delivery device 100. In these embodiments, the data may be used, in conjunction with data from temperature sensor 106, to determine whether a target object was a living being or an inanimate object.

In some embodiments, contact sensor 104 may include a plurality of sensors. For example, contact sensor 104 may include both a mechanical sensor and an electrical sensor described above.

The processes above are described as being performed by contact sensor 104 (e.g., determining whether there is contact, determining whether the contact is proper, activating/deactivating delivery apparatus 102, generating temperature context data). However, in some embodiments, the processes may be at least partially performed, or enabled, by one or more processors that may be internal and/or external to contact sensor 104. That is, one or more processors may be configured to perform the above-described processes at least partially and/or enable contact sensor 104 to perform them. Further, one or more of such processors may be shared among components of drug delivery device 100.

### Temperature Sensor

As discussed above, temperature sensor 106 may be capable of measuring ambient temperature, contact temperature, post-contact temperature. The terms "ambient temperature" refers to the temperature measured by temperature sensor 104 before contact sensor 104 comes in contact with an object. The terms "contact temperature" refers to the temperature measured by temperature sensor 106 while contact sensor 104 is in contact with an object. The terms "post-contact temperature" refers to the temperature measured by temperature sensor 106 after contact sensor 104 disengages with the object.

Temperature sensor 106 may be configured to detect temperature at a location near contact sensor 104. Accordingly, temperature sensor 106 may be capable of measuring temperature of an area of an object that comes in contact with contact sensor 104 ("contact area"). In some embodiments, temperature sensor 106 may be disposed adjacent, or substantially proximate, to contact sensor 104.

In some embodiments, temperature sensor 106 may be configured to make at least one measurement of ambient temperature. In some embodiments, temperature sensor 106 may be configured to make a plurality of measurements of ambient temperature. In some embodiments, temperature sensor 106 may be configured to make measurements of ambient temperature periodically and/or based on a predetermined timing scheme. For example, temperature sensor 106 may be configured to make a measurement of ambient temperature every 5 seconds. In some embodiments, measurement rate for ambient temperature may be dynamically determined based on a number of factors including, for example, battery level, rate of change in the recently measured ambient temperature, and/or variance of the recently measured ambient temperature. For example, temperature sensor 106 may increase the measurement rate for ambient temperature when the variance of the ambient temperature measured in the last few minutes are above a threshold variance. In another example, temperature sensor 106 may increase the measurement rate for ambient temperature when the last several measured ambient temperatures show a sharp increase/decrease. In yet another example, temperature sensor 106 may decrease the measurement rate for ambient temperature when drug delivery device 100 has less than, for example, ~10% battery level.

In some embodiments, temperature sensor 106 may make at least one measurement of contact temperature. In some embodiments, temperature sensor 106 may be configured to make a plurality of measurements of contact temperature. In some embodiments, temperature sensor 106 may be configured to make measurements of contact temperature periodically and/or based on a predetermined timing scheme. For example, temperature sensor 106 may be configured to make a measurement of contact temperature every 10 milliseconds. In some embodiments, measurement rate for contact temperature may be dynamically determined based on a number of factors including, for example, rate of change in the recently measured contact temperature, and/or variance of the measured contact temperature. For example, temperature sensor 106 may increase the measurement rate for contact temperature when the variance of the contact temperature measured in the last few measurements are above a threshold variance. In another example, temperature sensor 106 may increase the measurement rate for contact temperature when the last several measurements show a sharp increase/decrease. In some embodiments, a measurement rate of contact temperature may be higher than a measurement rate of ambient temperature.

In some embodiments, temperature sensor 106 may make at least one measurement of post-contact temperature. In some embodiments, temperature sensor 106 may be configured to make a plurality of measurements of post-contact temperature. As discussed above, the measured temperature may be considered post-contact temperature, as opposed to ambient temperature, for a predetermine amount of time after contact sensor 104 disengages a target object, until the temperature stabilizes (e.g., variance is below a threshold value), and/or until the measured temperature reaches, or become close to, a previously-measured ambient temperature or a predetermined temperature. In some embodiments, the post-contact temperature may include at least one measurement after the temperature stabilizes.

In some embodiments, temperature sensor 106 may be configured to make measurements of post-contact temperature periodically and/or based on a predetermined timing scheme. For example, temperature sensor 106 may be configured to make a measurement of post-contact temperature every 10 milliseconds. In some embodiments, measurement rate for post-contact temperature may be dynamically determined based on a number of factors including, for example, battery level, rate of change in the recently measured post-contact temperature, and/or variance of the measured post-contact temperature. For example, temperature sensor 106 may increase the measurement rate for post-contact temperature when the variance of the post-contact temperature measured in the last few minutes are above a threshold variance (e.g., 20%). In another example, temperature sensor 106 may increase the measurement rate for post-contact temperature when the last several measured post-contact temperatures show a sharp increase/decrease (e.g., 2F/sec). In yet another example, temperature sensor 106 may decrease the measurement rate for post-contact temperature when drug delivery device 100 has less than, for example, ~25% battery level.

In some embodiments, a measurement rate of post-contact temperature may be lower than a measurement rate of contact temperature. In some embodiments, a measurement rate/timing of post-contact temperature may be the same, or substantially the same, as a measurement rate/timing for ambient temperature.

The processes above are described as being performed by temperature sensor 106 (e.g., determining measurement timing/scheme, taking a measurement, identifying ambient/contact/post-contact temperature measurements). However, in some embodiments, the processes may be at least partially performed, or enabled, by one or more processors that may be internal and/or external to temperature sensor 106. That is, one or more processors may be configured to perform the above-described processes at least partially and/or enable temperature sensor 106 to perform them. Further, one or more of such processors may be shared among components of drug delivery device 100.

### Identifying Living Being / Inanimate Object

In the example of FIG. 1, drug delivery device 100, based on the measured temperatures (e.g., ambient temperature, contact temperature, and/or post-contact temperature), may determine whether a target object was a living being or an inanimate object. In some embodiments, drug delivery device 100 may determine whether a target object was a living being or an inanimate object further based on data generated by contact sensor 104 (e.g., raw sensor data or data indicative of whether contact sensor 104 is in contact with an object).

In some embodiments, drug delivery device 100 may determine whether a target object was a living being or an inanimate object further based temperature context data (generated by contact sensor 104 or one or more processors). As discussed above, temperature context data may include, for example, data that may be used to determine which portion of the temperature data is ambient temperature (i.e., temperature before contact sensor 104 is in contact with an object), which portion of the temperature data is contact temperature (i.e., temperature while contact sensor 104 is in contact with the object), and which portion of the temperature data is post-contact temperature (i.e., temperature when contact sensor 104 is no longer in contact with the object). In one example, the temperature context data may include time/date corresponding to when ambient temperature ends, contact temperature begins/ends, and/or when post-contact temperature begins/ends. In another example, temperature context data may identify temperature measurements corresponding to (i) the first and/or last ambient temperature measurements, (ii) the first and/or last contact temperature measurements, and/or (iii) the first and/or last post-contact temperature measurements.

In some embodiments, drug delivery device 100 may transmit the measured temperature to an external device, and the external device may analyze the measured temperature to determine whether a target object was a living being or an inanimate object. Alternatively, or additionally, drug delivery device 100 transmit the measured temperature to an external device, and the external device may partially analyze the measured temperature and transmit the result of the analysis back to drug delivery device 100. In response, drug delivery device 100 may complete the analysis to determine whether a target object was a living being or an inanimate object.

In some embodiments, drug delivery device 100 determines whether a target object of a dose was a living being or an inanimate object after each dose. Alternatively, drug delivery device 100 determine, in batch, whether target objects of a plurality of doses are a living being or an inanimate object.

In some embodiments, after the dose is delivered, drug delivery device 100 may record that a dose is delivered. In some embodiments, the record may include, for example, time/date when the dose was delivered, identification of the substance, identification of container 110, volume of the dose inside container 110, volume of the dose delivered while contact sensor 104 is activated. The record may further include whether the dose was likely delivered to a living being or an inanimate object. In some embodiments, drug delivery device 100 may transmit the record(s) to an external device (e.g., patient's mobile device, a cloud-based application, a "smart" case for device 100).

In some embodiments, drug delivery device 100, based on measured ambient temperature and/or post-contact temperature, calibrates the contact temperature prior to using the contact temperature to determine whether a target object was a living being or an inanimate object. In some embodiments, drug delivery device 100 may use a predetermined ratio(s) that quantitatively describes the relationship between (i) ambient and/or post-contact temperature and (ii) the contact temperature. For example, for every 1°F below 80°F that an ambient and/or post-contact temperature drops, the contact temperature may be expected to drop 0.25°F. In these embodiments, a contact temperature of 82°F would not register as a living being when the ambient and/or post-contact temperature was 75°F. But, a contact temperature of 82°F would be registered as a living being when the ambient and/or post-contact temperature was 60°F.

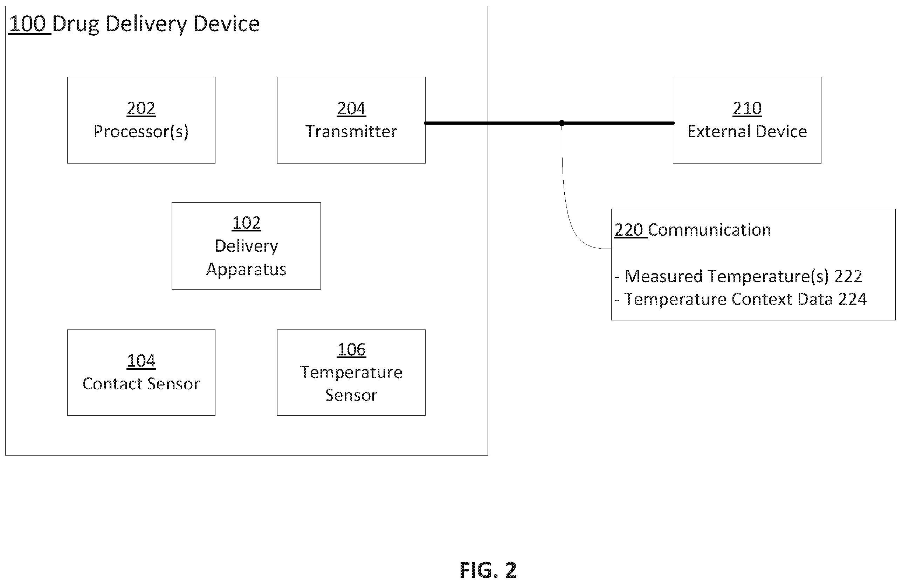

FIG. 2 illustrates various hardware components of drug delivery device 100 of FIG. 1. As discussed above, drug delivery device 100 includes delivery apparatus 102, contact sensor 104, and temperature sensor 106. As shown in FIG. 2, drug delivery device 100 may further include one or more processors 202, and a transmitter 204. Although not shown in FIG. 2, drug delivery device 100 may further include one of more of the following components: battery, receiver (or transceiver), RFID tag/transmitter (e.g., for identifying device 100, container 110, and/or delivery apparatus 102), a needle cap, LED display, motion sensor(s) (e.g. accelerometer, magnetometer, gyroscope), temperature sensor for measuring temperature of the substance in container 110.

As discussed above, processors 202 may be configured to at least partially perform and/or enable one or more functionalities of components such as delivery apparatus 102, contact sensor 104, and temperature sensor 106. Processors 202 may be further configured to transmit, using transmitter 204, a communication 220 to an external device 210. In some embodiments, one or more processors 202 may be a part of contact sensor 104, temperature sensor 106, and/or delivery apparatus 102. In some embodiments, processors 202 may include an analog and/or digital circuit for processing signal generated and/or received by contact sensor 104, temperature sensor 106, and/or delivery apparatus 102. For example, processors 202 may include thresholding circuit(s), signal conditioning circuit(s), and/or analog-to-digital and/or digital-to-analog converter(s).

In FIG. 2, communication 220 may include data that can be used by external device 210 to determine whether a target dose(s) is likely a living being or an inanimate object. In some embodiments, communication 220 may include measured temperature 222. For example, communication 220 may include contact temperature measured by temperature sensor 106. In another example, communication 220 may include contact temperature as well as ambient temperature and/or post-contact temperature measured by temperature sensor 106. In yet another example, communication 220 may include contact temperature as well as a stabilized portion of post-contact temperature measured by temperature sensor 106. In some embodiments, communication 220 may further include temperature context data 224. As discussed above, temperature context data 224 may be used to identify which portion of measured temperature 222 corresponds to ambient temperature, contact temperature, and/or post-contact temperature. In some embodiments, temperature context data 224 may identify which portion of measured temperature 222 corresponds to a stabilized portion of post-context temperature.

In some embodiments, each measurement included in measured temperature 222 may be associated with data (e.g., a timestamp) indicative of when the measurement was taken. In some embodiments, each measurement included in measured temperature 222 may be associated with data indicative of when the measurement was taken with respect to when delivery apparatus 102 is activated and/or deactivated.

In some embodiments, communication 220 may further include data generated by contact sensor 104 (e.g., data generated during contact, data that was used to determine that contact sensor 104 is or is not in contact with an object).

In some embodiments, communication 220 may further include data associated with one or more doses delivered by drug delivery device 100, such as a volume of a dose, identification of the substance, identification of a patient associated with device 100, and time/date when the dose was delivered.

Communication 220 may be transmitted (i) after a dose is delivered, (ii) after a predetermined number of doses have been delivered, (iii) based on a predetermined schedule, (iv) based on an input from a user (e.g., after a user presses a button on drug delivery device 100), and/or (v) based on a request from an external device (e.g., external device 210).

External device 210, based on data included in communication 220, may determine whether the target object to which a dose delivered by drug delivery device 100 was indeed a living human being. Alternatively, or additionally, external device 210 may partially analyze the measured temperature and transmit the result of the analysis back to drug delivery device 100. In response, drug delivery device 100 may complete the analysis to determine whether the target object was a living being or an inanimate object.

Transmitter 204 may include a wired and/or a wireless transmitter(s). For example, transmitter 204 may include a Bluetooth transmitter, a near-field communication (NFC) transmitter, or a cellular transmitter (e.g. LTE).

In some embodiments, external device 210 may be a mobile device, for example, operated by patient 120 or a healthcare professional. In some embodiments, external device 210 may be a dedicated device is paired with external device 210. In some embodiments, external device 210 may be capable of encasing drug delivery device 100 when not in use. In some embodiments, external device 210 may a implemented on a cloud platform.

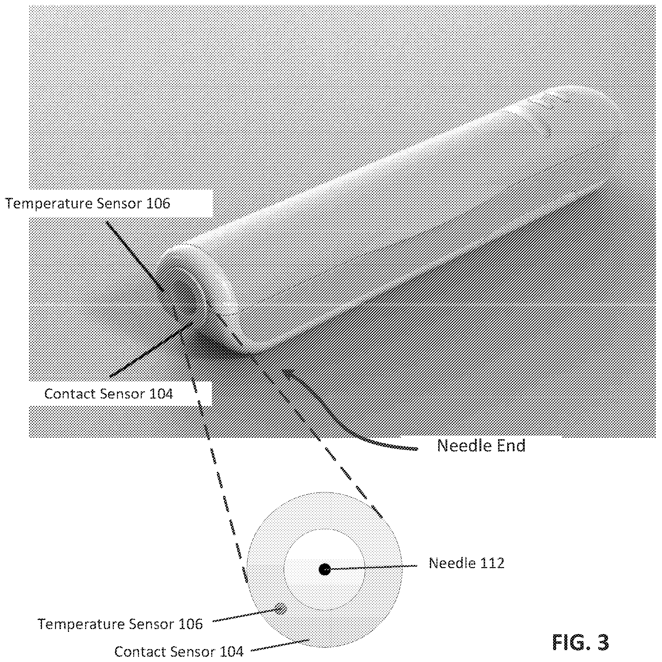

FIG. 3 illustrates an example of a drug delivery device 100 in accordance with the disclosed embodiments. As shown in FIG. 3, drug delivery device 100 may have a cylinder-like structure, and contact sensor 104 and temperature sensor 106 may be disposed on the needle-end of the cylinder-like structure. In particular, a sensing surface of contact sensor 104 may have a ring shape. Further, a sensing surface of temperature sensor 106 may be disposed within the ring-shaped sensing surface of contact sensor 104. In FIG. 3, needle 112 is in a retracted state; needle 112 may extend (e.g., after delivery apparatus 102 activates) through the center of the ring shape. In some embodiments, a sensing surface of temperature sensor 106 may have a ring shape, and a sensing surface of contact sensor 104 may be disposed within the sensing surface of the temperature sensor 106.

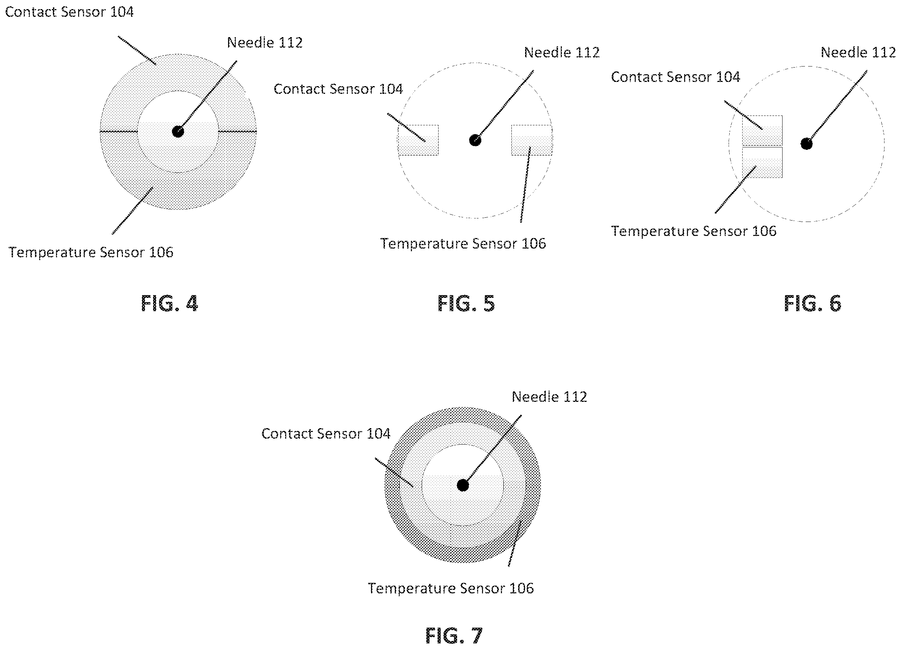

FIGS. 4-7 illustrate additional examples of arrangements for sensing surfaces in accordance with the disclosed embodiments. In FIG. 4, contact sensor 104 and temperature sensor 106, as a collective, may form a ring shape. A portion of the ring (e.g., half of the ring) may be a surface of contact sensor 104, and another portion of the ring may be a surface of temperature sensor 104. Needle 112 may be disposed, or extend through, the center of the ring. In FIG. 5, sensing surfaces of contact sensor 104 and temperature sensor 106 may be separated by a needle. Thus, in FIG. 5, a sensing surface of a contact sensor 104 may be disposed substantially proximate to a sensing surface of temperature sensor 106. In FIG. 6, sensing surfaces of contact sensor 104 and temperature sensor 106 may be adjacent to each other. The sensing surfaces may also be adjacent to needle 112. In FIG. 7, a sensing surface of a contact sensor 104 forms an inner ring while a sensing surface of temperature sensor 106 forms an outer ring. The centers of the rings may be aligned. Needle 112 may be disposed, or extend through, the center of both rings.

In some embodiments, contact sensor 104 may include a mechanical pressure sensor, and temperature sensor 106 may be arranged such that temperature sensor 106 comes in contact with an object after the mechanical pressure sensor 104 becomes compressed upon contact with the object. In some embodiments, contact sensor 104 and temperature sensor 106 may be arranged such that both sensors come in contact with an object substantially at the same time. For example, a sensing surface of contact sensor 104 and a sensing surface of temperature sensor 106 may be at substantially the same level.

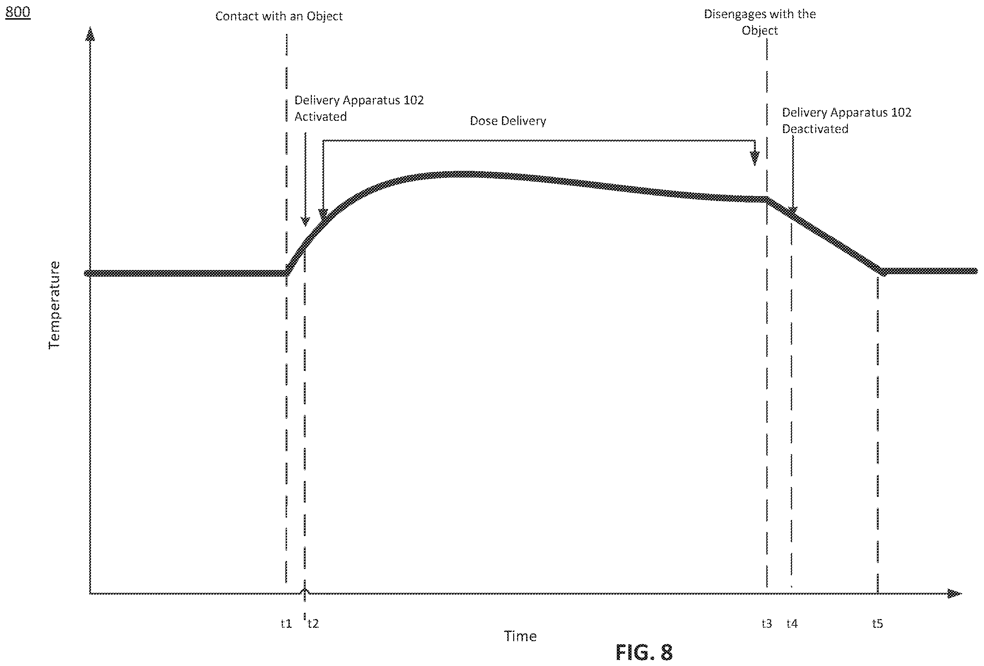

FIGS. 8-10 are example graphs illustrating temperature measured by temperature sensor 106 before, during, and after contact sensor 104 is in contact with an object.

FIG. 8 illustrates an example graph 800 illustrating temperature measured by temperature sensor 106 around a time period when drug delivery device 100 delivers a dose to a living patient 120. In FIG. 8, prior to t1, the measured temperature is steady (i.e., low variance) at room temperature. The temperature measured during this time may be considered ambient temperature.

At t1, contact sensor 104 and temperature sensor 106 come in contact with the abdomen of patient 120. Thus, the measured temperature begins to rapidly increase at an expected rate (e.g.,75°F to 92°F over 3 seconds).

At t2, delivery apparatus 102 is activated, and delivery apparatus 102 begins delivering a dose into the arm of patient 120. A delay between t1 and t2 may be due to the time taken for contact sensor 104 (or a processor) to determine that contact sensor 104 is indeed in contact with an object. For example, contact sensor 104 may wait until the measured pressure/conductivity stabilizes before determining that contact sensor 104 is in contact with an object. Alternatively, or additionally, there may be an intentional delay between t1 and t2, for example, to ensure that patient 120 is ready/emotionally ready for the injection. In some embodiments, t1 and t2 may be the same. That is, delivery apparatus 102 may be activated as soon as contact sensor 104 is in contact with an object. In between t2 and t3, the measured temperature may be affected primarily by two variables: temperature of the substance and ambient temperature. In the example of FIG. 8, the substance in container 110 is at room temperature. But, if the substance in container 110 was at refrigerated temperatures, the measured temperature may decrease at an expected rate as the injection site is cooled by the refrigerated substance. In the example of FIG. 8, one or more pieces of clothing covering the contact area (abdomen) have been removed immediately, and exposure to the ambient temperature decreases the measured temperature. At least for these reasons, the measured temperature may increase at a slower, expected rate and/or decrease at an expected rate while the dose is being delivered into patient (e.g., 92°F to 89°F over 10 seconds).

At t3, a dose is delivered and patient 120 may disengage drug delivery device 100 from the abdomen. Thus, the measured temperature begins to decrease.

At t4, contact sensor 104 determines that contact sensor 104 is no longer in contact with an object. Any delay between t3 and t4 may be due to the time taken for contact sensor 104 (or a processor) to determine that contact sensor 104 is no longer in contact with an object. For example, contact sensor 104 may wait until the measured pressure/conductivity stabilizes before determining that contact sensor 104 is no longer in contact with an object. In some embodiments, t3 and t4 may be the same. That is, delivery apparatus 102 may be deactivated immediately after contact sensor 104 disengages with the object.

At t5, the measured temperature may reach the previously measured ambient temperature prior to t1. In some embodiments, the measured temperature between t3 and t5 may be used to calibrate the measured temperature between t1/t2 and t3/t4. In some embodiments, the measured temperature after t5 may be used to calibrate the measured temperature between t1/t2 and t3/t4. In some embodiments, the measured temperature before t1 may be used to calibrate the measured temperature between t1/t2 and t3/t4. In some embodiments, calibration may involve using a predetermined ratio(s) that quantitatively describes the relationship between (i) ambient (t1) and/or post-contact (t5) temperature and (ii) the contact temperature (t1-t3). For example, for every 1F° below 80F° that an ambient and/or post-contact temperature drops, the contact temperature is expected to drop 0.25F°. Therefore, a contact temperature of 82F° would not register as a living being when the ambient and/or post-contact temperature was 75F°. But, a contact temperature of 82F° would be registered as a living being when the ambient and/or post-contact temperature was 60F°.

In the example of FIG. 8, drug delivery device 100 or an external device may determine that the target object was a living being because the rates of temperature increase and/or decrease after t2 were at expected rates. Moreover, ambient temperature measure prior to t1 or measured temperature after t5 may be used to calibrate the contact temperature (i.e., between t1/t2 and t3/t4).

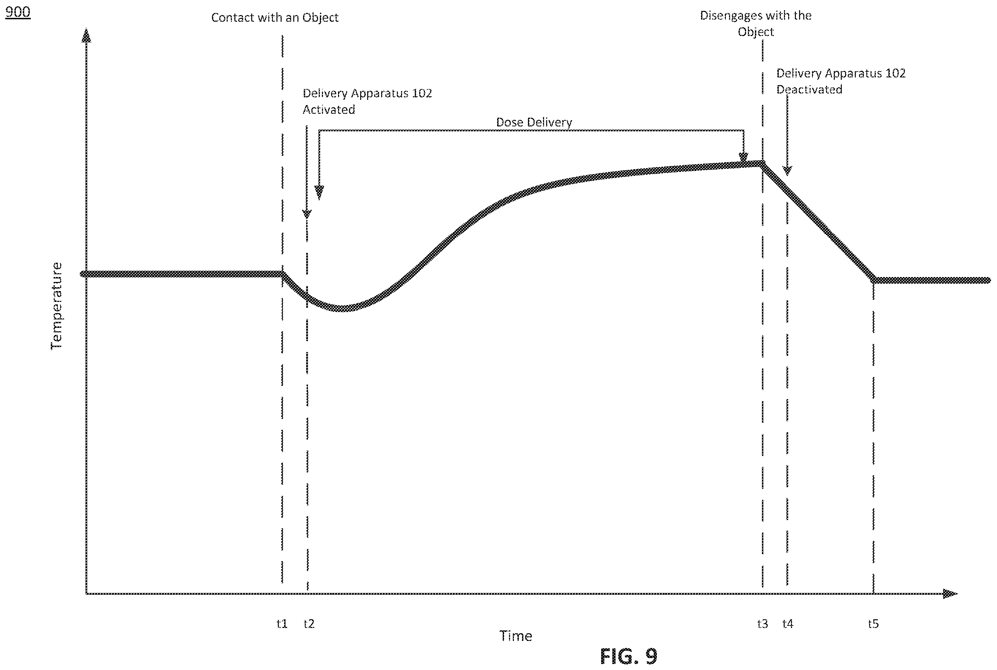

FIG. 9 illustrates another example graph 900 illustrating temperature measured by temperature sensor 106 around a time period when drug delivery device 100 delivers a dose to a living patient 120. FIG. 9 is similar to FIG. 8, except that patient 120 of FIG. 9 may have applied ice to the contact area before device 100/contact sensor 104 comes in contact with patient 120.

Accordingly, when contact sensor 104 and/or temperature sensor 106 come in contact with the abdomen of patient 120 at t1, the measured temperature begins to decrease at an expected rate for an iced contact (e.g., 75°F to 62°F over 3 seconds) as the temperature sensor reaches equilibrium with the temperature of the iced contact site. Furthermore, in contrast to the example of FIG. 8, the measured temperature then begins to increase at another expected rate as the iced area begins to warm (e.g., 62°F to 71°F over 15 seconds).

In the example of FIG. 9, drug delivery device 100 or an external device may determine that the target object was a living being and that the delivery area was an iced area because the rates of temperature decrease and subsequent increase after t1 were at expected rates for an iced area of a living being. The rate of the increase after t1 may be especially important for determining that the target object was a living being because body heat will cause the measured temperature of the contact site to increase faster than an inanimate object that was iced or taken out of the refrigerator.

The expected rates and/or values may be predetermined. For example, a vendor/manufacturer of drug delivery device 100 may conduct experiments to determine the expected rates and/or values. In some embodiments, the multiple set of expected rates and/or values may be determined, each set being associated with a particular injection site (e.g., arm, thigh, abdomen). In some embodiments, each set may be associated with a different body size and/or composition. For example, a set of expected rates and/or values may be determined for a person having a relatively high body fat percentage while another set of expected rates and/or values may be determined for a person having a relatively low body fat percentage. In some embodiments, a set of expected rates and/or values may be associated with a particular substance in container 110. For example, certain substances that may cause inflammation/increase in skin temperature may have a different set of expected rates and/or values than substances that do not cause inflammation/increase in skin temperature. In some embodiments, the expected rates and/or values may be associated with a particular person.

In some embodiments, the expected rates and/or values may be determined based on historical temperature measurements received from a plurality of drug delivery devices. In such embodiments, statistical analysis may be applied to define a series of expected rates and values. For example, drug delivery events that have a set of rates and values similar to FIG. 8 would be categorized as non-iced sites, and events that have a set of rates and values similar to FIG. 9 would be categorized as iced sites. In some embodiments, further statistical analysis would be applied to create a quantitative range of contact temperature over time, for each category. In some embodiments, statistical analysis is applied to create a quantitative range for specific timepoints, such as t1, or time ranges, such as t1-t3. By creating quantitative ranges, outliers and anomalies can be identified, and drug delivery events previously labeled as living being can be corrected and labeled as inanimate objects. In some embodiments, the quantitative ranges are created using calibrated contact temperature measurements, correcting for variance in ambient temperature. In some embodiments, the expected rates and/or values may be determined based on historical, temperature measurements received from a particular drug delivery device 100. In such embodiments, statistical analysis may be applied to create an intrapersonal quantitative range of contact temperature over time, in order to identify anomalies that differ from the patient's normal injection routine.

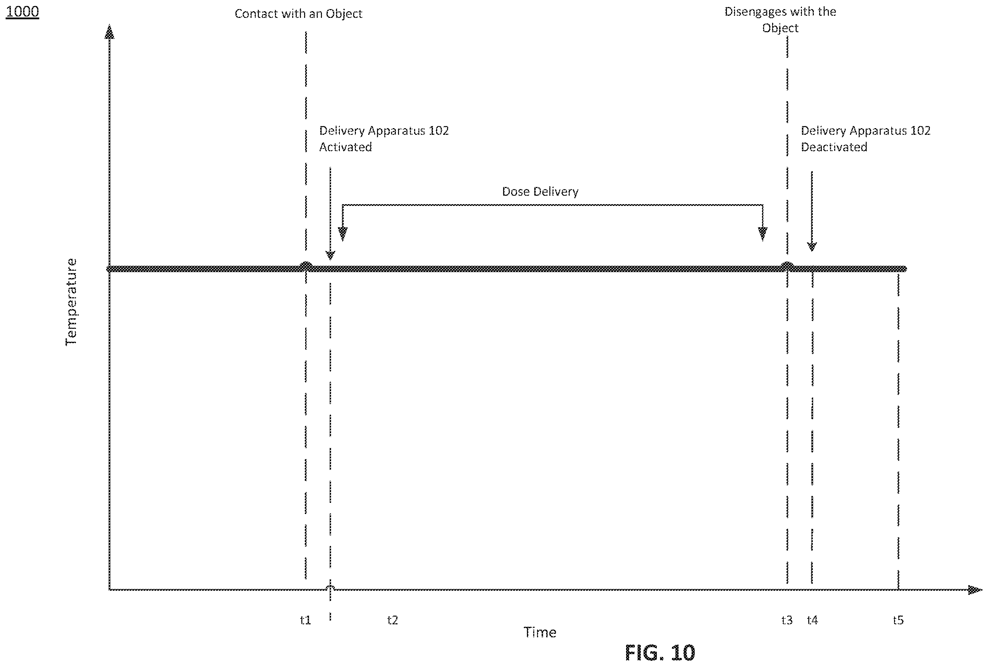

FIG. 10 illustrates yet another example graph illustrating temperature measure by temperature sensor 106 when drug delivery device 100 delivers a dose to an inanimate object (e.g., orange or wet paper towel). In FIG. 10, the measured temperature remains at, or substantially near, the ambient temperature. Thus, drug delivery device 100 or an external device may determine that the target object was an inanimate object because the rates of temperature change after t1 were not at the expected rates.

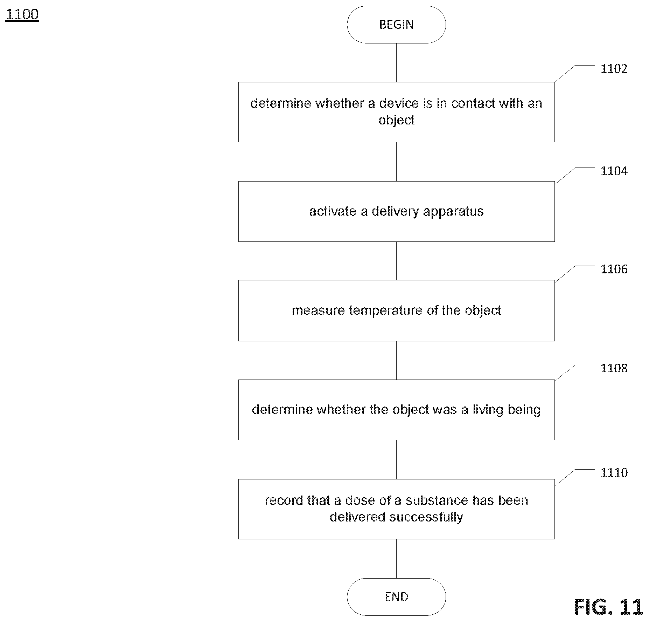

FIG. 11 illustrates an example of a process 1100 for delivering a dose of a substance to a living being by a drug delivery device 100 in accordance with the disclosed embodiments.

At a step 1102, contact sensor 104, or a processor using contact sensor 104, may determine whether the device is in contact with an object.

At a step 1104, contact sensor 104, or a processor using contact sensor 104, may activate delivery apparatus 102 after determining that the device 100 or a sensing surface of contact sensor 104 is in contact with the object. The activation of delivery apparatus 102 may cause delivery apparatus 102 to deliver a dose of the substance to the object, for example, by actuating actuator 108 to push on a plunger of a container 110 that include the substance.

At a step 1106, temperature sensor 106, or a processor using temperature sensor 106, may measure temperature of the object. In some embodiments, the measured temperature may include a plurality of measurements. In some embodiments, measuring of the temperature of the object may include measuring the temperature of the object periodically and/or based on a predetermined measurement timing scheme.

At an optional step, temperature sensor 106, or a processor using temperature sensor 106, may measure temperature before the delivery apparatus is activated and/or temperature after the delivery apparatus is deactivated. In some embodiments, the measured temperature of the object may be calibrated by the temperature before the delivery apparatus is activated and/or the temperature after the delivery apparatus is deactivated.

At an optional step, contact sensor 104, or a processor using contact sensor 104, may deactivate delivery apparatus 102 after determining that the device 100 or a sensing surface of contact sensor 104 is no longer in contact with the object

At an optional step, a transmitter, or a processor using the transmitter, may transmit a communication to an external device. The communication may include the measured temperature.

At a step 1108, the external device, in response to receiving the communication, may determine whether the object was a living being based on the measured temperature. Alternatively, a processor of drug delivery device 100 may determine whether the object was a living being based on the measured temperature. In some embodiments, the determination of whether the object was a living being may include comparing the measured temperature with data generated based on previously measured temperatures transmitted by one or more drug delivery devices or a particular drug delivery device. In some embodiments, the determination of whether the object was a living being may include comparing a rate of temperature change after the delivery apparatus is activated with an expected rate of temperature change. In some embodiments, the determination of whether the object was a living being may include determining whether the measured temperature changed at least by a predetermined amount after the delivery apparatus is activated.

At a step 1110, the external device or a processor of drug delivery device 100 may record that a dose of the substance has been delivered successfully after determining that the object was a living being.

At an optional step, contact sensor 104, delivery apparatus 102, and/or a processor of drug delivery device 100 may be configured to deactivate the delivery apparatus 102 after determining that the device 100 or a sensing surface of contact sensor 104 is no longer in contact with the object.

In some embodiments, delivery apparatus 102 may be configured to deliver the dose of the substance to a delivery site of the object. In these embodiments, the contact sensor, the temperature sensor, and the delivery apparatus may be substantially proximate to each other such that, when the delivery apparatus is activated, the contact sensor determines whether the contact sensor is in contact with the delivery site of the object and the temperature sensor measures temperature at the delivery site of the object.

From the foregoing, it will be appreciated that specific embodiments of the invention have been described herein for purposes of illustration, but that various modifications may be made without deviating from the scope of the invention. Accordingly, the invention is not limited except as by the appended claims.

## Claims

1. A system comprising an external device (210) and a device (100) for delivering a dose of a substance to a living being, the device (100) for delivering a dose of the substance comprising:
a contact sensor (104) positioned such that the contact sensor (104) is in contact with an object when the device (100) is in a proper position for delivering a dose of the substance to the object;
a delivery apparatus (102) configured to deliver a dose of the substance to the object after the delivery apparatus (102) is activated;
a temperature sensor (106) configured to measure temperature of the object after the delivery apparatus (102) is activated;
a transmitter (204) configured to communicate with the external device (210); and
one or more processors (202) configured to:
determine, using the contact sensor (104), whether the contact sensor (104) is in contact with the object;
activate the delivery apparatus (102) after determining that the contact sensor (104) is in contact with the object, wherein the delivery apparatus (102), after being activated, delivers a dose of the substance to the object;
measure, using the temperature sensor (106), contact temperature of the object after the delivery apparatus (102) is activated;
transmit, using the transmitter (204), a communication to the external device (210), wherein the communication includes the measured temperature (222); and
**characterized in that** the external device (210), in response to receiving the communication,
(i) determines, based on the measured temperature (222), whether the object was a living being, and
(ii) records that the substance has been delivered successfully to a living being after determining that the object was a living being
wherein the measured temperature includes a plurality of measurements,
and wherein the determination of whether the object was a living being includes comparing a rate of temperature change after the delivery apparatus (102) is activated with an expected rate of temperature change.

2. The system of claim 1, wherein the processors are further configured to measure, using the temperature sensor (106), ambient temperature before the delivery apparatus (102) is activated and/or ambient temperature after the delivery apparatus (102) is deactivated.

3. The system or device of claim 2, wherein the processors are further configured to calibrate the measured temperature based on the temperature before the delivery apparatus (102) is activated and/or temperature after the delivery apparatus (102) is deactivated.

4. The system of claim 1, wherein the processors are further configured to deactivate the delivery apparatus (102) after determining that the contact sensor (104) is no longer in contact with the object.

5. The system of claim 1, wherein the determination of whether the object was a living being includes determining whether the measured temperature changed at least by a predetermined amount after the delivery apparatus (102) is activated.

6. The system of claim 1, wherein the delivery apparatus (102) is configured to deliver the dose of the substance to a delivery site of the object, and wherein the contact sensor (104), the temperature sensor (106), and the delivery apparatus (102) are substantially proximate to each other such that, when the delivery apparatus (102) is activated, the contact sensor (104) determines whether the contact sensor (104) is in contact with the delivery site of the object and the temperature sensor (106) measures temperature at the delivery site of the object.

7. The system of claim 1, wherein the measuring of the temperature includes measuring the temperature periodically or based on a predetermined measurement timing scheme.

8. The system of claim 1, wherein the determination of whether the object was a living being includes comparing the measured temperature with data generated based on previously measured temperatures provided by one or more delivery apparatus.

9. The system of claim 1, wherein the determination of whether the object was a living being includes comparing, after the delivery apparatus (102) is activated, a temperature value and a temperature change with an expected temperature value and temperature change, respectively.

10. The system of claim 1, wherein the determination of whether the object was a living being includes determining whether the measured temperature was within a predetermined range of values and whether the measured temperature changed at a predetermined rate after the delivery apparatus (102) is activated.

11. The system or device of claim 10, wherein the predetermined rate can be a negative rate or a positive rate.

## Patentansprüche

1. System, umfassend eine externe Vorrichtung (210) und eine Vorrichtung (100) zum Abgeben einer Dosis einer Substanz an ein Lebewesen, die Vorrichtung (100) zum Abgeben einer Dosis der Substanz umfassend:
einen Kontaktsensor (104), der derart positioniert ist, dass der Kontaktsensor (104) in Kontakt mit einem Objekt steht, wenn sich die Vorrichtung (100) in einer ordnungsgemäßen Position zum Abgeben einer Dosis der Substanz an das Objekt befindet;
eine Abgabeeinrichtung (102), die konfiguriert ist, um eine Dosis der Substanz an das Objekt abzugeben, nachdem die Abgabeeinrichtung (102) aktiviert wurde;
einen Temperatursensor (106), der konfiguriert ist, um die Temperatur des Objekts zu messen, nachdem die Abgabeeinrichtung (102) aktiviert wurde;
einen Sender (204), der konfiguriert ist, um mit der externen Vorrichtung (210) zu kommunizieren; und
ein oder mehrere Prozessoren (202), die konfiguriert sind zum:
Bestimmen, unter Verwendung des Kontaktsensors (104), ob der Kontaktsensor (104) in Kontakt mit dem Objekt steht;
Aktivieren der Abgabeeinrichtung (102), nachdem bestimmt wurde, dass der Kontaktsensor (104) in Kontakt mit dem Objekt steht, wobei die Abgabeeinrichtung (102), nachdem sie aktiviert wurde, eine Dosis der Substanz an das Objekt abgibt;
Messen, unter Verwendung des Temperatursensors (106), der Kontakttemperatur des Objekts, nachdem die Abgabeeinrichtung (102) aktiviert wurde;
Übertragen, unter Verwendung des Senders (204), einer Kommunikation an die externe Vorrichtung (210), wobei die Kommunikation die gemessene Temperatur (222) einschließt; und
**dadurch gekennzeichnet, dass** die externe Vorrichtung (210) als Reaktion auf das Empfangen der Kommunikation,
(i) basierend auf der gemessenen Temperatur (222), bestimmt, ob das Objekt ein Lebewesen war, und
(ii) verzeichnet, dass die Substanz erfolgreich an ein Lebewesen abgegeben wurde, nachdem bestimmt wurde, dass das Objekt ein Lebewesen war
wobei die gemessene Temperatur eine Vielzahl von Messungen einschließt,
und wobei die Bestimmung, ob das Objekt ein Lebewesen war, ein Vergleichen einer Temperaturänderungsrate, nachdem die Abgabeeinrichtung (102) aktiviert wurde, mit einer erwarteten Temperaturänderungsrate einschließt.

2. System nach Anspruch 1, wobei die Prozessoren ferner konfiguriert sind, um, unter Verwendung des Temperatursensors (106), eine Umgebungstemperatur, bevor die Abgabeeinrichtung (102) aktiviert wird, und/oder die Umgebungstemperatur, nachdem die Abgabeeinrichtung (102) deaktiviert wurde, zu messen.

3. System oder Vorrichtung nach Anspruch 2, wobei die Prozessoren ferner konfiguriert sind, um die gemessene Temperatur basierend auf der Temperatur, bevor die Abgabeeinrichtung (102) aktiviert wird, und/oder der Temperatur, nachdem die Abgabeeinrichtung (102) deaktiviert wurde, zu kalibrieren.

4. System nach Anspruch 1, wobei die Prozessoren ferner konfiguriert sind, um die Abgabeeinrichtung (102) zu deaktivieren, nachdem bestimmt wurde, dass der Kontaktsensor (104) nicht länger in Kontakt mit dem Objekt steht.

5. System nach Anspruch 1, wobei die Bestimmung, ob das Objekt ein Lebewesen war, das Bestimmen einschließt, ob sich die gemessene Temperatur um mindestens einen vorbestimmten Betrag geändert hat, nachdem die Abgabeeinrichtung (102) aktiviert wurde.

6. System nach Anspruch 1, wobei die Abgabeeinrichtung (102) konfiguriert ist, um die Dosis der Substanz an eine Abgabestelle des Objekts abzugeben, und wobei der Kontaktsensor (104), der Temperatursensor (106) und die Abgabeeinrichtung (102) im Wesentlichen nahe beieinander derart sind, dass, wenn die Abgabeeinrichtung (102) aktiviert wird, der Kontaktsensor (104) bestimmt, ob der Kontaktsensor (104) in Kontakt mit der Abgabestelle des Objekts steht, und der Temperatursensor (106) die Temperatur an der Abgabestelle des Objekts misst.

7. System nach Anspruch 1, wobei das Messen der Temperatur ein periodisches Messen der Temperatur einschließt oder basierend auf einem vorbestimmten Messzeitschema erfolgt.

8. System nach Anspruch 1, wobei die Bestimmung, ob das Objekt ein Lebewesen war, das Vergleichen der gemessenen Temperatur mit Daten einschließt, die basierend auf zuvor gemessenen Temperaturen erzeugt werden, die durch eine oder mehrere Abgabeeinrichtungen bereitgestellt werden.

9. System nach Anspruch 1, wobei die Bestimmung, ob das Objekt ein Lebewesen war, das Vergleichen, nachdem die Abgabeeinrichtung (102) aktiviert wurde, eines Temperaturwerts und einer Temperaturänderung mit einem erwarteten Temperaturwert beziehungsweise einer erwarteten Temperaturänderung einschließt.

10. System nach Anspruch 1, wobei die Bestimmung, ob das Objekt ein Lebewesen war, das Bestimmen einschließt, ob die gemessene Temperatur innerhalb eines vorbestimmten Wertebereichs lag und ob sich die gemessene Temperatur mit einer vorbestimmten Rate änderte, nachdem die Abgabeeinrichtung (102) aktiviert wurde.

11. System oder die Vorrichtung nach Anspruch 10, wobei die vorbestimmte Rate eine negative Rate oder eine positive Rate sein kann.

## Revendications

1. Système comprenant un dispositif externe (210) et un dispositif (100) permettant de délivrer une dose d'une substance à un être vivant, le dispositif (100) permettant de délivrer une dose de la substance comprenant :
un capteur de contact (104) positionné de telle sorte que le capteur de contact (104) est en contact avec un objet lorsque le dispositif (100) est dans une position adéquate permettant de délivrer une dose de la substance à l'objet ;
un appareil de délivrance (102) conçu pour délivrer une dose de la substance à l'objet après que l'appareil de délivrance (102) a été activé ;
un capteur de température (106) configuré pour mesurer la température de l'objet après que l'appareil de délivrance (102) a été activé ;
un transmetteur (204) configuré pour communiquer avec le dispositif externe (210) ; et
un ou plusieurs processeurs (202) configurés pour :
déterminer, à l'aide du capteur de contact (104), si le capteur de contact (104) est en contact avec l'objet ;
activer l'appareil de délivrance (102) après détermination que le capteur de contact (104) est en contact avec l'objet, dans lequel l'appareil de délivrance (102), après avoir été activé, délivre une dose de la substance à l'objet ;
mesurer, à l'aide du capteur de température (106), une température de contact de l'objet après que l'appareil de délivrance (102) a été activé ;
transmettre, à l'aide du transmetteur (204), une communication au dispositif externe (210), dans lequel la communication comporte la température mesurée (222) ; et
**caractérisé en ce que** le dispositif externe (210), en réponse à la réception de la communication,
(i) détermine, en fonction de la température mesurée (222), si l'objet était un être vivant, et
(ii) enregistre que la substance a été délivrée avec succès à un être vivant après détermination que l'objet était un être vivant
dans lequel la température mesurée comporte une pluralité de mesures,
et dans lequel la détermination quant à savoir si l'objet était un être vivant comporte la comparaison d'un taux de changement de température après que l'appareil de délivrance (102) a été activé à un taux de changement de température attendu.

2. Système selon la revendication 1, dans lequel les processeurs sont configurés en outre pour mesurer, à l'aide du capteur de température (106), la température ambiante avant que l'appareil de délivrance (102) ne soit activé et/ou la température ambiante après que l'appareil de délivrance (102) a été désactivé.

3. Système ou dispositif selon la revendication 2, dans lequel les processeurs sont configurés en outre pour étalonner la température mesurée en fonction de la température avant que l'appareil de délivrance (102) ne soit activé et/ou de la température après que l'appareil de délivrance (102) a été désactivé.

4. Système selon la revendication 1, dans lequel les processeurs sont configurés en outre pour désactiver l'appareil de délivrance (102) après détermination que le capteur de contact (104) n'est plus en contact avec l'objet.

5. Système selon la revendication 1, dans lequel la détermination quant à savoir si l'objet était un être vivant comporte le fait de déterminer si la température mesurée a changé au moins d'une quantité prédéterminée après que l'appareil de délivrance (102) a été activé.

6. Système selon la revendication 1, dans lequel l'appareil de délivrance (102) est conçu pour délivrer la dose de la substance à un site de délivrance de l'objet, et dans lequel le capteur de contact (104), le capteur de température (106) et l'appareil de délivrance (102) sont sensiblement à proximité les uns des autres de telle sorte que, lorsque l'appareil de délivrance (102) est activé, le capteur de contact (104) détermine si le capteur de contact (104) est en contact avec le site de délivrance de l'objet et le capteur de température (106) mesure la température au niveau du site de délivrance de l'objet.

7. Système selon la revendication 1, dans lequel la mesure de la température comporte la mesure de la température périodiquement ou en fonction d'un schéma de temporisation de mesure prédéterminé.

8. Système selon la revendication 1, dans lequel la détermination quant à savoir si l'objet était un être vivant comporte la comparaison de la température mesurée à des données générées en fonction de températures précédemment mesurées fournies par un ou plusieurs appareils de délivrance.

9. Système selon la revendication 1, dans lequel la détermination quant à savoir si l'objet était un être vivant comporte la comparaison, après que l'appareil de délivrance (102) a été activé, d'une valeur de température et d'un changement de température à une valeur de température et à un changement de température attendus, respectivement.

10. Système selon la revendication 1, dans lequel la détermination quant à savoir si l'objet était un être vivant comporte le fait de déterminer si la température mesurée était au sein d'une plage prédéterminée de valeurs et si la température mesurée a changé à un taux prédéterminé après que l'appareil de délivrance (102) a été activé.

11. Système ou dispositif selon la revendication 10, dans lequel le taux prédéterminé peut être un taux négatif ou un taux positif.
